# EUROPÄISCHE PATENTSCHRIFT

(11) **EP 0 547 727 B1**
(45) Veröffentlichungstag und Bekanntmachung des Hinweises auf die Patenterteilung: **13.03.1996**
(21) Anmeldenummer: 92250353.7
(22) Anmeldetag: 04.12.1992
(51) Int. Cl.: A61K 7/48, A01N 31/02

(54) **Hautantiseptikum und Händedesinfektionsmittel**
Skin and hands composition containing disinfectants and antiseptics
Composition pour la peau et les mains contenant des agents désinfectants et antiseptiques

(30) Priorität: 09.12.1991 DE 4140473
(43) Veröffentlichungstag der Anmeldung: 23.06.1993
(73) Patentinhaber: SCHÜLKE & MAYR GMBH, D-22840 Norderstedt (DE)
(72) Erfinder: Diehl, Karl-Heinz, W-2000 Norderstedt (DE); Goroncy-Bermes, Peter, Dr., W-2070 Ahrensburg (DE); Eggensperger, Heinz, Dr., W-2000 Hamburg 65 (DE); Oltmanns, Peter, Dr., W-2000 Hamburg 20 (DE); Töfke, Susanne, Dr., W-2000 Hamburg 13 (DE)
(74) Vertreter: UEXKÜLL & STOLBERG

(56) Entgegenhaltungen:
- EP-A- 0 373 661
- EP-A- 0 375 827
- DE-A- 3 017 221

## Beschreibung

Die Erfindung betrifft Zusammensetzungen, die als Hautantiseptikum und Händedesinfektionsmittel verwendet werden können.

Seit langem werden Haut- oder Schleimhautpartien vor operativen Eingriffen, Injektionen oder Punktionen sowie vor Inspektionen von von außen zugänglichen Hohlorganen antiseptisch behandelt. Außerdem ist es notwendig, daß auch diejenigen Personen, die die vorgenannten Maßnahmen durchführen, ihre Hände vor Beginn des Eingriffs oder der Untersuchung desinfizieren.

Für die genannten Zwecke werden bisher Zusammensetzungen verwendet, deren antiseptisch wirkenden Bestandteile leicht flüchtige Alkohole sind, denn es ist bekannt, daß Alkohole eine gute Wirkung gegen Mikroorganismen haben und die Wirkung auf der Haut relativ schnell eintritt. Allerdings müssen für die Wirksamkeit innerhalb einer sehr kurzen Einwirkzeit (innerhalb von Sekunden bis zu wenigen Minuten) hohe Alkoholkonzentrationen vorliegen. Der Alkoholgehalt beträgt im allgemeinen mehr als 50, meistens etwa 60 bis 80% Gew.%.

Die in diesen Lösungen enthaltenen Alkohole sind häufig aliphatische Alkohole wie Ethanol, 1-Propanol und 2-Propanol.

In Händedesinfektionsmitteln sind neben derartigen Alkoholen meistens auch noch andere Substanzen wie Langzeitwirkstoffe, z.B. kationenaktive Verbindungen mit mikrobieller Wirkung, und um einer starken Entfettung der Haut vorzubeugen, hautpflegende Komponenten enthalten. Bei rückfettende Verbindungen enthaltenden Hautantiseptika muß der Alkoholgehalt häufig höher sein als bei Präparaten ohne Rückfetter, da diese Verbindungen oft die antiseptische Wirksamkeit der Alkohole oder anderer vorhandener mikrobieller Wirkstoffe beeinträchtigen.

Trotz der rückfettenden Hautpflegekomponenten kommt es in den kälteren Jahreszeiten bei der notwendigerweise häufigen Anwendung dieser Präparate vermehrt zu Irritationen der damit behandelten Haut.

Neben alkoholischen Hautantiseptika und Händedesinfektionsmitteln sind ferner antimikrobiell und desinfizierende wäßrige Emulsionen bekannt, die als Wirkstoff einen Glycerinmonoalkylether, z.B. 3-Alkoxy-1,2-propandiol, enthalten (beispielsweise bekannt aus DE 649 206). Die antimikrobielle Wirksamkeit der Glycerinether (z.B. auch aus JP 76-76424 bekannt) bei deren alleinigen Verwendung hat sich in der Praxis jedoch als relativ gering erwiesen, so daß ihr Einsatz als Wirkstoff in erhebliche Mengen Wasser enthaltenden Hautantiseptika ohne weitere ebenfalls antimikrobiell wirkende Zusätze völlig unzureichend ist.

EP-A-0 375 827 betrifft Hautdesinfektionsmitteln oder Antibakterienmitteln, die Ethanol, Polyoxyethylen Glycerin und Wasser enthalten.

Der Erfindung liegt daher die Aufgabe zugrunde, ein besonders hautverträgliches, nachhaltig rückfettendes und damit hautpflegendes und trotzdem schnell wirkendes Hautantiseptikum und Händedesinfektionsmittel zur Verfügung zu stellen, das weder in seiner Wirkung noch in seinen Anwendungseigenschaften durch Hautpflegekomponenten beeinträchtigt ist und darüber hinaus ein angenehmes Hautgefühl vermittelt.

Diese Aufgabe wird durch eine Zusammensetzung gemäß Anspruch 1 gelöst, die eine Alkylalkoholkomponente, mindestens einen Glycerinmonoalkylether und Wasser enthält.

Bevorzugte Ausführungsformen der erfindungsgemäßen Zusammensetzung sind Gegenstand der Unteransprüche.

Erfindungsgemäß wurde gefunden, daß eine Kombination aus einer Alkylalkoholkomponente und mindestens einem Glycerinmonoalkylether in wäßriger Lösung eine hervorragende antimikrobielle Wirksamkeit zeigt und gleichzeitig den reduzierten Fettgehalt der Haut wieder ergänzt.

Dies ist insbesondere deshalb überraschend, weil die Glycerinmonoalkylether selbst nur eine unzureichende mikrobielle Wirkung zeigen und ihre rückfettenden Eigenschaften bisher unbekannt sind.

Die antimikrobielle Wirksamkeit der erfindungsgemäßen Kombination ist größer als die der jeweiligen Alkylalkoholkomponenten oder Glycerinmonoalkylether allein. Es liegt daher ein echter Synergismus vor.

Aufgrund des synergistischen Effekts der Alkylalkoholkomponente und des Glycerinmonoalkylethers ist es darüberhinaus möglich, den Alkylalkoholgehalt der Zusammensetzung im Vergleich zu dem der bekannten als Hautantiseptika und Händedesinfektionsmittel verwendeten Zusammensetzungen zu senken.

Die Kombination von Alkylalkoholen in geringen Konzentrationen mit einem Glycerinmonoalkylether bietet daher nicht nur ökonomische Vorteile durch Senkung des Alkoholgehalts, sondern auch die Möglichkeit, die lokale Verträglichkeit von Hautantiseptika und Händedesinfektionsmitteln zu verbessern. Das Risiko einer Hautirritation ist daher insbesondere bei häufigem Gebrauch aufgrund der geringeren Entfettung und besseren Rückfettung erheblich vermindert.

Durch den erfindungsgemäßen Zusatz von Glycerinmonoalkylether in geringen Mengen zu einer Alkylalkoholkomponente ist es nun gelungen, eine ausreichend hohe Wirksamkeit und schnellen Wirkungseintritt sowie eine ausgesprochen gute Rückfettung zu erreichen.

Bei den Glycerinmonoalkylethern handelt es sich insbesondere um 1-(C₅-C₁₂-Alkyl)glycerinether. Hierzu gehören unter anderem 1-(2-Ethylhexyl)glycerinether, 1-Heptylglycerinether, 1-Octylglycerinether, 1-Decylglycerinether und 1-Dodecylglycerinether. Eine bevorzugte Verbindung ist der 1-(2-Ethylhexyl)glycerinether. Diese Verbindungen sind leicht zugänglich. Ihre Herstellung ist in der Literatur beschrieben, z.B. in JP 80-19253, JP 58-134049, DE 33 43 530 und E. Baer, H.O.L. Fischer in J. Biol. Chem. 140 397 (1941).

Als Alkylalkoholkomponente enthält die erfindungsgemäße Zusammensetzung mindestens einen aliphatischen C₁-C₆-Alkylalkohol. Insbesondere eignen sich hierfür aliphatischen Alkylalkohole wie Ethanol, 1-Propanol, 2-Propanol oder ein Gemisch aus zwei oder mehr dieser Alkohole.

Erfindungsgemäße Zusammensetzungen enthalten im allgemeinen 15 bis 85 Gew.%, insbesondere 20 bis 50 Gew.%, vorzugsweise 25 bis 40 Gew.% und bevorzugter 30 bis 35 Gew.% Alkylalkoholkomponente, 0,1 bis 5 Gew.%, vorzugsweise 0,5 bis 2,5 Gew.% und bevorzugter 0,5 bis 1,5 Gew.% Glycerinmonoalkylether und Wasser. Besonders bevorzugt enthält die Zusammensetzung 35 Gew.% Alkylalkoholkomponente, 1 Gew.% Glycerinmonoalkylether und Wasser.

Außerdem kann sie eine oder mehrere andere antiseptisch wirkende Verbindungen, Farbstoff und/oder Parfüm sowie andere gebräuchliche Additive und Hilfsstoffe, wie z.B. waschaktive Substanzen enthalten. Im Gegensatz zu den in bekannten Haut-antiseptika und Händedesinfektionsmitteln gegebenenfalls vorhandenen Additiven wie Hautpflegeadditiven beeinträchtigen in der erfindungsgemäßen Zusammensetzung vorhandene weitere Additive die antiseptische Wirkung nicht. Dadurch wird auch der Nachteil vermieden, bei Anwesenheit derartiger Additive wie beispielsweise hautpflegender Komponenten die Alkylalkoholkonzentration heraufsetzen zu müssen.

Die folgenden Beispiele 1 bis 6 zeigen mögliche Formulierungen für erfindungsgemäße Zusammensetzungen:

### Beispiel 1

| | |
|---|---|
| Ethanol | 85 % |
| o-Phenylphenol | 0,1 % |
| 1-(2-Ethylhexyl)glycerinether | 1,5 % |
| demineralisiertes Wasser | ad 100 % |
| Farbstoff | |
| Parfüm | |

### Beispiel 2

| | |
|---|---|
| 1-Propanol | 50 % |
| 2-Propanol | 20 % |
| 1-(2-Ethylhexyl)glycerinether | 1 % |
| demineralisiertes Wasser | ad 100 % |
| Farbstoff | |
| Parfüm | |

### Beispiel 3

| | |
|---|---|
| 2-Propanol | 70 % |
| Ethanol | 10 % |
| 1-Heptylglycerinether | 1,3 % |
| demineralisiertes Wasser | ad 100 % |
| Farbstoff | |
| Parfüm | |

### Beispiel 4

| | |
|---|---|
| Ethanol | 45 % |
| Benzethoniumchlorid | 0,6 % |
| 1-Decylglycerinether | 0,5 % |
| demineralisiertes Wasser | ad 100 % |
| Farbstoff | |
| Parfüm | |

### Beispiel 5

| | |
|---|---|
| 2-Propanol | 35 % |
| Milchsäure | 0,3 % |
| 1-Octylglycerinether | 0,8 % |
| demineralisiertes Wasser | ad 100 % |
| Farbstoff | |
| Parfüm | |

### Beispiel 6

| | |
|---|---|
| 1-Propanol | 55 % |
| Ethanol | 15 % |
| 1-(2-Ethylhexyl)glycerinether | 1,2 % |
| demineralisiertes Wasser | ad 100 % |
| Farbstoff | |
| Parfüm | |

Wie bereits erwähnt, weisen Alkylalkohole eine gute Wirkung gegen Mikroorganismen auf, wobei der Wirkungseintritt relativ kurzfristig (wenige Minuten) eintritt, aber um sehr kurze Einwirkzeiten (1 Minute oder weniger) zu erreichen, hohe Konzentrationen gewählt werden müssen.

In einem quantitativen Suspensionsversuch wurde die Beziehung zwischen der Einwirkdauer und der Alkoholkonzentration mit und ohne Glycerinmonoalkylether ermittelt. Wie die Ergebnisse in Tabelle I zeigen, wurde für eine Keimzahlreduktion von Pseudomonas aeruginosa um mehr als 5 log-Stufen bei einer Alkoholkonzentration von 35 Gew.% eine Einwirkzeit von 120 Sekunden benötigt bzw. wurde zur Halbierung dieser Einwirkdauer auf 60 Sekunden oder weniger mindestens eine Konzentration von 40% Ethanol benötigt. Demgegenüber reicht bei Verwendung einer erfindungsgemäßen Zusammensetzung, z.B. Zusatz eines Glycerinmonoalkylethers in geringen Mengen (1 Gew.%) zu 35%-igem Ethanol bereits eine Einwirkzeit von 60 Sekunden aus, um die gleiche Keimzahlreduktion zu erhalten. Bei gleicher Alkoholkonzentration konnte daher die Einwirkdauer auf die Hälfte reduziert werden bzw. konnte andersherum betrachtet der Ethanolgehalt bei gleicher Einwirkdauer um 5 Gew.% (eine Verringerung von 12,5 %) verringert werden.

**Tabelle I**

| **Keimzahlreduktion von S. aureus und P. aeruginosa im quantitativen Suspensionsversuch** | | | | |
|---|---|---|---|---|
| Ethanol-Konzentration in % | Einwirkzeit in s | | | |
| | 15 | 30 | 60 | 120 |
| P. aeruginosa | | | | |
| 20 | 0 | 0 | 0 | 0 |
| 30 | 0 | 0 | 0 | 0 |
| 35 | 1,05 | 1,60 | 2,55 | ≧5,48 |
| 40 | ≧5,49 | ≧5,43 | ≧5,40 | ≧5,48 |
| 35 % Ethanol + 1 % Glycerinether | | | ≧5,38 | |

| S. aureus | | | | |
|---|---|---|---|---|
| 35 | 0 | 0 | 0 | 1,16 |
| 40 | 0 | 1,11 | 2,04 | 5,09 |
| 35 % Ethanol + 1 % Glycerinether | | | ≧5,44 | |

Für die Reduktion der Keimzahl von Staphylococcus aureus um 5 log-Stufen war selbst bei Verwendung von 40%-igem Ethanol eine Einwirkdauer von 120 Sekunden erforderlich. Die gleiche Keimzahlreduktion konnte wiederum mit 35%-igem Ethanol in Kombination mit 1% Glycerinether in 60 Sekunden erreicht werden. In diesem Fall konnte also sowohl eine Verkürzung der Einwirkzeit als auch eine Verringerung der Alkoholkonzentration erreicht werden.

Durch Versuche auf der Haut der Unterarme von freiwilligen Probanden konnten die Ergebnisse der obigen quantitativen Suspensionsversuche bestätigt werden. Tabelle II zeigt die Reduktionsfaktoren, die mit 70%-igem Isopropanol (Referenzlösung gemäß DGHM-Richtlinie für die Prüfung und Bewertung von Hautdesinfektionsmitteln, Stand 1.1.91, Zbl. Hyg. 192 (1991), S. 99-103) bzw. 35%-igem Ethanol und mit einer Kombination von 35%-igem Ethanol und 1% Glycerinmonoalkylether nach 30, 60 und 120 Sekunden Einwirkdauer erzielt wurden. Die Ergebnisse zeigen, daß eine Kombination aus Ethanol und Ethylhexylglycerinether eine Wirkung hervorruft, die einerseits über das zu erwartende Ausmaß hinausgeht und andererseits trotz eines auf die Hälfte reduzierten Alkoholgehaltes mit der Wirkung von 70%-igem Isopropanol vergleichbar ist. Da die Glycerinether allein nur in untergeordnetem Ausmaß mikrobiell wirkt, ist von einer synergistischen Wirkung auszugehen.

**Tabelle II**

| **Keimzahlreduktion von Micrococcus luteus auf der Haut** | |
|---|---|
| Einwirkzeit: 30 s | RF |
| 70 % Isopropanol | 2,11 |
| 35 % Ethanol | 0,37 |
| 35 % Ethanol + 1 % Ethylhexylglycerinether | 1,62 |

| Einwirkzeit: 60 s | |
|---|---|
| 70 % Isopropanol | 2,30 |
| 35 % Ethanol | 0,61 |
| 35 % Ethanol + 1 % Ethylhexylglycerinether | 1,71 |

| Einwirkzeit : 120 s | |
|---|---|
| 70 % Isopropanol | 2,37 |
| 35 % Ethanol | 0,65 |
| 35 % Ethanol + 1 % Ethylhexylglycerinether | 1,79 |

Glycerinmonoalkylether als Reinsubstanzen sind ohne Alkoholzusatz in Wasser schwer löslich. Wäßrige Suspensionen dieser Ether zeigen eine für die angegebenen Zwecke unzureichende mikrobielle Wirkung.

Die oben genannten Versuche gemäß Tabelle I und II wurden mit den Wirkstoffen Ethanol und Isopropanol als Einzelsubstanzen und der Kombination Ethanol-Glycerinether in wäßriger Lösung getestet. Die zur Überprüfung der mikrobiziden Wirksamkeit verwendeten Testorganismen waren Staphylococcus aureus, Pseudomonas aeruginosa und Micrococcus luteus. Die Bestimmung der bakteriziden Wirkung erfolgte im quantitativen Suspensionsversuch nach den Richtlinien der Deutschen Gesellschaft für Hygiene und Mikrobiologie (DGHM) und im Hautversuch nach Williamson und Kligmann.

Ein besonderer Nutzen des Einsatzes von Glycerinether ergibt sich daraus, daß er insbesondere in den niedrigen Konzentrationen, in denen er zur Steigerung der antimikrobiellen Wirksamkeit beiträgt, herausragende rückfettende Eigenschaften zeigt. Dies ist ganz im Sinne moderner Formulierungen, bei denen aus Gründen der besseren Verträglichkeit möglichst wenig verschiedene Inhaltsstoffe eingesetzt werden sollen.

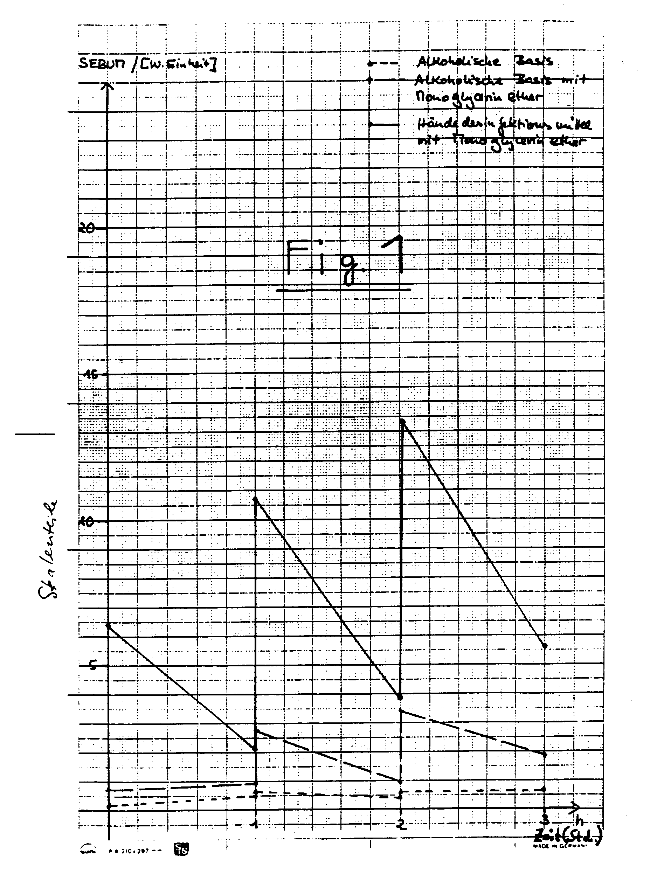

In Hautmessungen konnte gezeigt werden, daß der Glycerinether bei einer Anwendung über den Tag eine leichte Aufkonzentration des Fettgehalts auf der Haut bewirkt, ohne daß sich dies negativ als Schmierfilm bemerkbar macht (siehe Figur 1).

Als Meßgerät wurde hierbei ein Sebumeter SM 810 PC von Courage und Khazaka, Köln verwendet. Die nicht vorbehandelten Hände wurden stündlich mit jeweils 3 ml Händedesinfektionsmittel 90 Sekunden lang eingerieben. Nach 3 Stunden wird der Versuch abgebrochen. Die Messungen wurden direkt vor und nach jeder Anwendung durchgeführt, wobei von jeder Hand zweimal der Handrücken und einmal die Handinnenfläche gemessen wurden (Hautfettmessungen).

Zur Demonstration der gegenüber bekannten Händedesinfektionsmiteln überlegenen rückfettenden Eigenschaft der erfindungsgemäßen Glycerinether enthaltenden Zusammensetzungen wurden Messungen des Hautfettgehaltes mit dem Sebumeter durchgeführt. Die Meßbedingungen und das Gerät waren die gleichen wie oben.

Es wurden folgende Formulierungen vergleichend untersucht:

### Formulierung A

| | |
|---|---|
| 60 % | Ethanol |
| 20 % | i-Propanol |
| ad 100 % | Wasser |

### Formulierung B

| | |
|---|---|
| 60 % | Ethanol |
| 20 % | i-Propanol |
| 1 % | 1-(2-Ethylhexyl)glycerinether |
| ad 100 % | Wasser |

### Formulierung C

| | |
|---|---|
| 60 % | Ethanol |
| 20 % | i-Propanol |
| 1 % | Myristylalkohol |
| ad 100 % | Wasser |

### Formulierung D

| | |
|---|---|
| 60 % | Ethanol |
| 20 % | i-Propanol |
| 1 % | Myristylalkohol |
| 1 % | 1-(2-Ethylhexyl)glycerinether |
| ad 100 % | Wasser |

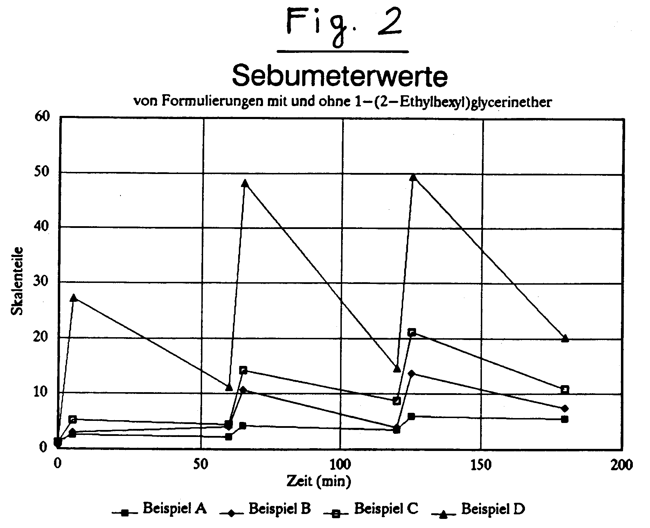

Die graphische Auftragung der erhaltenen Sebumeter-Werte (siehe Figur 2) verdeutlicht die überlegene durch den Glycerinether hervorgerufene rückfettende Wirkung. Um einen besseren Vergleich zu den bisher bekannten Händedesinfektionsmitteln zu erhalten, wurde in allen Formulierungen ein Gesamtalkoholgehalt von 80% gewählt, der in handelsüblichen Präparaten gängig ist.

Nach Anwendung von Formulierung B verbleibt im Vergleich zur Anwendung von Formulierung A, wobei der Unterschied zwischen den Formulierungen darin besteht, daß Formulierung A keinen Glycerinether enthält, nach jeder Anwendung ein höherer Hautfettgehalt.

Besonders deutlich wird dieser Effekt bei einem Vergleich der Formulierungen C und D. Mit Formulierung D werden deutlich höhere Hautfettgehalte erreicht als mit Formulierung C, die bereits eine bekannte Rückfettungskomponente (Myristylalkohol) enthält. Der höhere Hautfettgehalt, der nach Anwendung der Formulierung D erhalten wird, nimmt zwar innerhalb einer Stunde nach erfolgter Anwendung deutlich ab, es verbleibt jedoch, insbesondere nach mehrfacher Anwendung ein höherer Fettgehalt als nach Anwendung der Formulierung C.

Subjektiv wahrnehmbar ist die verstärkt rückfettende Eigenschaft der Glycerinether in den erfindungsgemäßen Zusammenzungen durch ein nach der Anwendung verbleibendes angenehmes Hautgefühl, welches mit den Adjektiven samtig, geschmeidig, glatt, nicht-fettend und nicht-klebrig beschrieben werden kann.

Die erfindungsgemäßen Zusammensetzungen können hergestellt werden, indem die Einzelkomponenten gegebenenfalls unter Erwärmen nacheinander miteinander vermischt werden. Eine bestimmte Reihenfolge braucht hierbei nicht eingehalten zu werden.

Die wünschenswerte Kombination aus hervorragender biozider bzw. biostatischer, mikrobieller Wirkung und erheblich verbesserter rückfettender Wirkung ist nur möglich, wenn in dem jeweiligen Präparat die erfindungsgemäße Zusammensetzung mit der Kombination von Alkylkomponente mit Glycerinmonoalkylether vorhanden ist, wobei eine der Komponenten, hier der Glycerinmonoalkylether, ein Additiv ist, das für die beabsichtigten Zwecke keine ausreichende Eigenwirkung als Antiseptikum oder Desinfektionsmittel hat, die Wirksamkeit aber auf synergistische Weise steigert. Darüberhinaus ist gleichzeitig eine Verringerung des Wirkstoffgehalts möglich, wodurch die Fettentfernung von der Haut herabgestzt wird.

Die erfindungsgemäßen Zusammensetzungen sind daher insgesamt gesehen als Hautantiseptika und Händedesinfektionsmittel vorzüglich geeignet.

## Patentansprüche

1. Verwendung einer Zusammensetzung, die eine Alkylalkoholkomponente, mindestens einen Glycerinmonoalkylether und Wasser enthält, zur Herstellung eines Hautantiseptikums und Händedesinfektionsmittels.

2. Verwendung nach Anspruch 1, dadurch gekennzeichnet, daß der Glycerinmonoalkylether ein 1-(C₅-C₁₂-Alkyl)glycerinether und insbesondere ausgewählt ist aus 1-(2-Ethylhexyl)glycerinether, 1-Heptylglycerinether, 1-Octylglycerinether, 1-Decylglycerinether oder 1-Dodecylglycerinether ist.

3. Verwendung nach Anspruch 1 oder 2, dadurch gekennzeichnet, daß die Alkylalkoholkomponente einen aliphatischen C₁₋C₆₋ Alkylalkohol, insbesondere ausgewählt aus Ethanol, 1-Propanol, 2-Propanol oder einem Gemisch aus zwei oder mehr dieser Alkohole enthält.

4. Verwendung nach einem der Ansprüche 1 bis 3, dadurch gekennzeichnet, daß sie außerdem eine oder mehrere andere antiseptisch wirkende Verbindungen, Hilfsstoffe wie waschaktive Substanzen, Farbstoff und/oder Parfüm enthält.

5. Verwendung nach einem der Ansprüch 1 bis 4, dadurch gekennzeichnet, daß die Zusammensetzung
15 - 85 Gew.% Alkylalkoholkomponente
0,1 - 5 Gew.% Glycerinmonoalkylether und Wasser,
insbesondere
20 - 50 Gew.% Alkylalkoholkomponente und
0,5 - 2,5 Gew.% Glycerinmonoalkylether,
vorzugsweise
25 - 40 Gew.% Alkylalkoholkomponente und
0,5 - 1,5 Gew.% Glycerinmonoalkylether,
bevorzugter
30 - 35 Gew.% Alkylalkoholkomponente und
1 Gew.% Glycerinmonoalkylether
und speziell bevorzugt
35 Gew.% Alkylalkoholkomponente und/oder
1 Gew.% Glycerinmonoalkylether
enthält.

6. Zusammensetzung dadurch gekennzeichnet, daß sie eine Alkylalkoholkomponente, mindestens einen Glycerinmonoalkylether und Wasser enthält, wobei der Glycerinmonoalkylether ausgewählt ist aus 1-(2-Ethylhexyl)glycerinether, 1-Heptylglycerinether oder 1-Octylglycerinether.

7. Zusammensetzung nach Anspruch 6, dadurch gekennzeichnet, daß die Alkylalkoholkomponente einen aliphatischen C₁-C₆-Alkylalkohol ausgewählt aus Ethanol, 1-Propanol, 2-Propanol oder einem Gemisch aus zwei oder mehr dieser Alkohole enthält.

8. Zusammensetzung nach einem der Ansprüche 6 oder 7, dadurch gekennzeichnet, daß sie außerdem eine oder mehrere andere antiseptisch wirkende Verbindungen, Hilfsstoffe wie waschaktive Substanzen, Farbstoff und/oder Parfüm enthält.

9. Zusammensetzung nach einem der Ansprüche 6 bis 8, dadurch gekennzeichnet, daß sie
15 - 85 Gew.% Alkylalkoholkomponente
0,1 - 5 Gew.% Glycerinmonoalkylether und Wasser,
insbesondere
20 - 50 Gew.% Alkylalkoholkomponente und
0,5 - 2,5 Gew.% Glycerinmonoalkylether,
vorzugsweise
25 - 40 Gew.% Alkylalkoholkomponente und
0,5 - 1,5 Gew.% Glycerinmonoalkylether,
bevorzugter
30 - 35 Gew.% Alkylalkoholkomponente und
1 Gew.% Glycerinmonoalkylether
und speziell bevorzugt
35 Gew.% Alkylalkoholkomponente und/oder
1 Gew.% Glycerinmonoalkylether
enthält.

## Claims

1. Use of a composition, which contains one alkyl alcohol component, at least one glycerine monoalkyl ether and water, for producing a skin antiseptic and hand disinfectant.

2. Use according to claim 1, characterized in that the glycerine monoalkyl ether is a 1-(C₅₋C₁₂ alkyl) glycerine ether and in particular is selected from 1-(2-ethylhexyl) glycerine ether, 1-heptyl glycerine ether, 1-octyl glycerine ether, 1-decyl glycerine ether or 1-dodecyl glycerine ether.

3. Use according to claim 1 or 2, characterized in that the alkyl alcohol component contains an aliphatic C₁₋C₆ alkyl alcohol, in particular selected from ethanol, 1-propanol, 2-propanol or a mixture of two or more of these alcohols.

4. Use according to one of claims 1 to 3, characterized in that it further contains one or more other antiseptically acting compounds, auxiliaries such as washing active substances, dye and/or perfume.

5. Use according to one of claims 1 to 4, characterized in that the composition contains
15 - 85 % by wt. alkyl alcohol component
0.1 - 5 % by wt. glycerine monoalkyl ether and water,
in particular
20 - 50 % by wt. alkyl alcohol component and
0.5 - 2.5 % by wt. glycerine monoalkyl ether,
preferably
25 - 40 % by wt. alkyl alcohol component and
0.5 - 1.5 % by wt. glycerine monoalkyl ether,
more preferably
30 - 35 % by wt. alkyl alcohol component and
1 % by wt. glycerine monoalkyl ether
and particularly preferably
35 % by wt. alkyl alcohol component and/or
1 % by wt. glycerine monoalkyl ether.

6. Composition characterized in that it contains an alkyl alcohol component, at least one glycerin monoalkyl ether and water, whereby the glycerine monoalkyl ether is selected from 1-(2-ethylhexyl) glycerine ether, 1-heptyl glycerine ether or 1-octyl glycerine ether.

7. Composition according to Claim 6, characterized in that the alkyl alcohol component contains an aliphatic C₁-C₆ alkyl alcohol selected from ethanol, 1-propanol, 2-propanol or a mixture of two or more of these alcohols.

8. Composition according to one of claims 6 or 7, characterized in that it also contains one or more other antiseptically acting compounds, auxiliaries such as washing active substances, dye and/or perfume.

9. Composition according to one of claims 6 to 8, characterized in that it contains
15 - 85 % by wt. alkyl alcohol component
0.1 - 5 % by wt. glycerine monoalkyl ether and water,
in particular
20 - 50 % by wt. alkyl alcohol component and
0.5 - 2.5 % by wt. glycerine monoalkyl ether,
preferably
25 - 40 % by wt. alkyl alcohol component and
0.5 - 1.5 % by wt. glycerine monoalkyl ether,
more preferably
30 - 35 % by wt. alkyl alcohol component and
1 % by wt. glycerine monoalkyl ether
and particularly preferably
35 % by wt. alkyl alcohol component and/or
1 % by wt. glycerine monoalkyl ether.

## Revendications

1. Utilisation d'une composition contenant un composant alcanolique, au moins un monoalkyléther de glycéryle et de l'eau pour la préparation d'un antiseptique dermique et d'un agent de désinfection des mains.

2. Utilisation selon la revendication 1, caractérisée en ce que le monoalkyléther de glycéryle est un 1-(C₅-C₁₂ alkyl)glycéryl-éther et-il est notamment choisi parmi I-(2-éthylhexyl)glycéryl-éther, 1-heptylglycéryl-éther, 1-octylglycéryl-éther, 1-décylglycéryl-éther ou 1 -dodécylglycéryl-éther.

3. Utilisation selon la revendication 1 ou 2, caractérisée en ce que le composant alcanolique comprend un alcanol aliphatique en C₁ à C₆ choisi notamment parmi éthanol, propanol-1, propanol-2, ou un mélange de deux ou de plus de deux de ces alcools.

4. Utilisation selon l'une quelconque des revendications 1 à 3, caractérisée en ce que la composition contient en outre un ou plusieurs autres dérivés à action antiseptique, adjuvants, tels que substance activant le lavage, colorant et/ou parfum.

5. Utilisation selon l'une quelconque des revendications 1 à 4, caractérisée en ce que la composition comprend:
- 15 à 85 % de poids de composant alcanolique
- 0,1 à 5 % de poids de monoalkyléther de glycéryle
- et de l'eau
notamment
- 20 à 50 % en poids de composant alcanolique et
- 0,5 à 2,5% en poids de monoalkyléther de glycéryle,
de préférence
- 25 à 40 % en poids de composant alcanolique et
- 0,5 à 1,5 % en poids de monoalkyléther de glycéryle,
de préférence
- 30 à 35 % en poids de composant alcanolique et
- 1 % en poids de monoalkyléther de glycéryle
et tout particulièrement de préférence
- 36 % en poids de composant alcanolique et/ou
- 1 % en poids de monoalkyléther de glycéryle.

6. Composition caractérisée en ce qu'elle comprend un composant alcanolique, au moins un monoalkyléther de glycéryle et de l'eau, le monoalkyléther de glycéryle étant choisi parmi 1-(2-éthylhexyl)glycéryl-éther, 1-heptylglycéryl-éther ou 1-octylglycéryl-éther.

7. Composition selon la revendication 6, caractérisée en ce que le composant alcanolique comprend: un alcanol aliphatique en C₁ à C₆ choisi parmi l'éthanol, le propanol-1, le propanol-2 ou un mélange de deux ou de plus de deux de ces alcools.

8. Composition selon l'une quelconque des revendications 6 ou 7, caractérisée en ce qu'elle comprend en outre un ou plusieurs autres dérivés à effet antiseptique, adjuvants, tels que substances activante du lavage, colorant et/ou parfum.

9. Composition selon l'une quelconque des revendications 6 à 8 caractérisée en ce qu'elle comprend:
- 15 à 85 % de poids de composant alcanolique
- 0,1 à 5 % de poids de monoalkyléther de glycéryle
- et de l'eau
notamment
- 20 à 50 % en poids de composant alcanolique et
- 0,5 à 2,5% en poids de monoalkyléther de glycéryle,
de préférence
- 25 à 40 % en poids de composant alcanolique et
- 0,5 à 1,5 % en poids de monoalkyléther de glycéryle,
de préférence
- 30 à 35 % en poids de composant alcanolique et
- 1 % en poids de monoalkyléther de glycéryle
et tout particulièrement de préférence
- 35 % en poids de composant alcanolique et/ou
- 1 % en poids de monoalkyléther de glycéryle.
